# FASCICULE DE BREVET EUROPEEN

(11) **EP 0 605 700 B1**
(45) Date de publication et mention de la délivrance du brevet: **17.05.2000**
(21) Numéro de dépôt: 93916037.0
(22) Date de dépôt: 26.07.1993
(51) Int. Cl.: A61K 31/475

(54) **COMPOSITION PHARMACEUTIQUE A BASE DE FLAVOPEREIRINE ET SON UTILISATION DANS UN TRAITEMENT CONTRE LE VIRUS VIH**
FLAVOPEREIRINE ENTHALTENDE ZUSAMMENSETZUNG UND IHRE ANWENDUNG IN DER BEHANDLUNG GEGEN HIV-VIREN
FLAVOPEREIRINE-BASED PHARMACEUTICAL COMPOSITION AND USE THEREOF FOR TREATING HIV

(30) Priorité: 28.07.1992 FR 9209284
(43) Date de publication de la demande: 13.07.1994
(73) Titulaire: Molecular International Research, Inc., New York, NY 10022-6501 (US)
(72) Inventeur: Beljanski, Mirko, 75005 Paris (FR)
(74) Mandataire: Leboyer, Jean-Jacques
(86) Numéro de dépôt international: FR9300761
(87) Numéro de publication internationale: WO9402146

(56) Documents cités:
- EP-A- 0 059 817
- EP-A- 0 352 147
- EP-A- 0 373 986
- CH-A- 679 982
- FR-A- 2 450 607
- EXP. CELL BIOL. vol. 50, no. 2, 1982, pages 79 - 87 BELJANSKI M. ET AL. 'SELECTIVE INHIBITION OF IN VITRO SYNTHESIS OF CANCER DNA BY ALKALOIDS OF BETA-CARBOLINE CLASS'
- J. AM. CHEM. SOC. vol. 80, 1958, pages 1601 - 1609 H. RAPOPORT ET AL. 'ALKALOIDS OF GEISSOSPERMUM VELOSII' cité dans la demande

## Description

La présente invention concerne l'utilisation antivirale de la flavopéreirine. Elle concerne plus particulièrement une composition pharmaceutique contenant comme seul principe actif de la flavopéreirine et l'utilisation de celle-ci pour le traitement chez l'homme des infections virales, en particulier telles que celles provoquées par le virus de l'immunodéficience humaine VIH.

La flavopéreirine est un alcaloïde de la classe des bêta-carbolines. Elle est également dénommée classiquement "composé H ou PB 100" et présente une fluorescence UV d'émission à 250-254 et 306 nm.

On peut l'obtenir à partir de l'écorce de Pao Pereira, Geissospermum vellosii-Baillon, Apocynaceae [voir H. Rapoport et al. (J. Am. Chem. Soc. 80, 1601-1608 (1958); et M. Beljanski et J. Bugiel: demande de 1^{er} certificat d'addition No. FR 2450607 à la demande de brevet français No. FR 2419725 et document EP-A-0 059 817].

Il est connu que la flavopéreirine, administrée à la dose de 200-600 µg par voie intradermique ou de 2,5-500 mg/jour, de préférence 30 mg/jour, empêche l'apparition et le développement des papules virales dans le cas de virus du type de ceux du fibrome de Shope et de la vaccine.

On sait également que la flavopéreirine est censée agir in vivo contre le virus de l'influenza (virus à ARN) et qu'elle peut en outre inhiber la multiplication du virus de la mosaïque du tabac (VMT) lors d'un bref contact avec ce virus.

Le document EP-A-0 059 817 divulgue en outre que la flavopéreirine est active contre le virus de la grippe, mais que la demi-vie d'une telle bêta-carboline quaternaire est trop courte pour qu'elle puisse être administrée efficacement chez l'homme sous une forme galénique autre que des microgranules à effet retard.

Le document FR 2639830 décrit quant à lui un système d'amélioration de la défense immunitaire chez les humains (contre les virus à ARN -SIDA en particulier- et les virus à ADN). Selon ce document l'inhibition de la multiplication des virus concernés n'est possible que par un système de quatre substances différentes, dont la flavopéreirine n'est que l'une d'entre elles. La composition pharmaceutique ainsi divulguée comprend obligatoirement au moins un représentant de chacune de ces quatre catégories de substances actives. La flavopéreirine comprise dans un tel système composite est administrée à une dose de 0,25 à 1 g/jour, de préférence par voie orale.

On a maintenant trouvé de façon inattendue que la flavopéreirine peut constituer à elle seule un principe actif efficace dans la lutte contre les virus de VIH chez les mammifères, y compris l'homme. On a plus particulièrement trouvé que la flavopéreirine est un principe actif qui à lui seul exerce, tant in vitro que in vivo, une action d'inhibition sélective sur l'infection virale par VIH, notamment chez des patients infectés par VIH-1.

La présente invention a donc pour d'objet l'utilisation d'une composition pharmaceutique pour l'obtention d'un médicament destiné au traitement des infections virales par VIH, comportant de la flavopéreirine comme unique principe actif, avantageusement sous une forme solide contenant par dose unitaire environ 500 mg de flavopéreirine ou d'un de ses sels ou autres dérivés pharmacologiquement acceptables.

Cette invention ressortira mieux de la suite de la présente description, dans laquelle les essais et expérimentations relatés ne sont donnés qu'à titre indicatif et ne limitent aucunement l'invention.

La flavopéreirine est dénuée d'effet toxique et d'effets secondaires chez les mammifères, y compris l'homme. La DL₅₀ chez le rat Sprague-Dawley EOPS est de 10,45 g/kg (limites de confiance: 9,63 à 11,35) par voie orale et de 2,45 g/kg (limites de confiance: 2,35 à 2,55) pour une administration intrapéritonéale. L'animal meurt dans les 30 à 60 minutes suivant l'administration orale ou intrapéritonéale, par arrêt respiratoire. Il n'a pas été observé de mort différée durant les 14 jours suivants.

Une administration sous-chronique par voie orale chez des rats Sprague-Dawley mâles et femelles (OFA) a démontré l'absence de toxicité à des doses égales à 1/20 de la DL₅₀ (soit 530 mg.kg⁻¹.d⁻¹) ou à 1/5 de la DL₅₀ (soit 2120 mg.kg⁻¹.d⁻¹). A ces doses on n'a détecté de modifications ni dans le poids corporel, ni dans la prise de nourriture. On n'a pas non plus décelé d'altération de la numération globulaire sanguine et les fonctions hépatiques et rénales restaient normales. On n'a observé au microscope aucune lésion du foie, des reins, du duodénum, du myocarde, de la rate, des glandes thyroïdes et parathyroïdes, des testicules et des ovaires.

La flavopéreirine franchit la barrière hématoencéphalique, ainsi que l'a montré le fait que chez des souris mâles CDI ayant reçu 10 mg de ce composé par voie orale, la teneur encéphalique en flavopéreirine était de 7 µg environ (soit une concentration de 14 µg/g, pour des souris de 20 g ayant un cerveau pesant 0,5 g).

Dans la pratique on prépare la flavopéreirine, en vue de son utilisation selon la présente invention, par exemple par hydrolyse dans HCl 1N de poudre de Geissospermum vellosii à 100°C, suivie d'une neutralisation par KOH, d'une extraction à l'éthanol et d'une réduction par distillation. On reprend ensuite le résidu de la distillation par du chloroforme, on élimine l'excès de sels par précipitation à froid en présence d'éthanol et on concentre le résidu, qui contient essentiellement la flavopéreirine.

Aux fins de la présente invention, la flavopéreirine peut être mise en oeuvre soit telle quelle, soit sous forme d'un sel ou autre dérivé pharmacologiquement acceptable.

Les essais pharmacologiques suivants, correspondant à l'indication thérapeutique susdite, ont été réalisés. Ils sont complétés par les figures annexées, dans lesquelles:
- Fig. 1 représente un graphique montrant les comptages comparés de cellules/ml en fonction du temps en heures suivant l'infection, l'addition de flavopéreirine (dénommée "H") étant faite 12 heures après l'infection;
- Fig. 2 représente les titrages comparés des unités infectieuses (en UI/ml) en fonction du temps en heures suivant l'infection, sans addition de composé H et avec addition de celui-ci respectivement à raison de 30 et 60 µg/ml.
- Fig. 3 représente sous forme de diagrammes l'effet du produit H (flavopèreirine) sur la production d'interleukine-6 par des monocytes humains provenant de donneurs sains.
- Fig. 4 représente sous forme de diagrammes l'effet du produit H sur la production spontanée d'interleukine-6 par des monocytes humains provenant de patients séropositifs.

### Effet antiviral contre VIH de la flavopéreirine

La destruction du VIH dans des cellules en culture in vitro sans effet sur les cellules normales ou saines a été démontrée par l'utilisation de la souche H-9 des lymphocytes T4 obtenue auprès du Dr. R. Gallo via le Paul Ehrlich Institute (Francfort, Allemagne) et propagée à l'Institut für Medizinische Mikrobiologie und Hygiene (Université de Berne, Suisse).

Le VIH avait été obtenu en tant que HTLV III auprès du Dr. Gallo et propagé dans les cellules H-9 susmentionnées. Le surnageant provenant de la culture de cellules H-9 infectées a été stocké à -80°C. Ce surnageant avait une concentration de 10⁶ UI/ml lorsqu'on l'utilisait. Les cellules H-9 ont été cultivées dans un milieu RPMI 1640 contenant 15% de sérum de veau foetal, 0,002 µm de glutamine et 100 UI de pénicilline/ml dans des fioles de Falcon (25 cm²). On a utilisé 8 à 20 ml de milieu par fiole. Les fioles ont été mises à incuber à 37°C, en position droite. La culture a été débutée à une concentration de 2 x 10⁵ cellules H-9 par ml et scindée lorsque le nombre de cellules atteignait 1 x 10⁶ cellules/ml. Pour tester la flavopéreirine dans des cultures de cellules H-9 infectées et non infectées, on a utilisé des plaques de microtitrage. Dans ce cas les cultures ont été débutées à une concentration de 6 x 10⁵ cellules/ml. Après 23 heures d'incubation il a été ajouté, à chaque alvéole contenant 0,1 ml de cette suspension cellulaire, 0,1 ml de milieu RPMI 1640 contenant 10² UI de VIH. Cela correspondait à une multiplicité d'infection de 1,6 x 10⁴, soit une unité infectieuse pour 500 cellules. 16 heures après l'infection, on a ajouté dans les alvéoles infectées et dans les alvéoles non infectées 0,1 ml de milieu RPMI 1640 avec ou sans flavopéreirine. La flavopéreirine a été utilisée à la concentration de 30 µg et de 60 µg/ml. Les plaques de microtitrage ont été recouvertes avec un couvercle de plaques d'Amersham et on a incubé à 37°C. Les comptages cellulaires ont été déterminés dans une chambre de Neubauer. On a déterminé la quantité totale d'antigène de VIH en utilisant un kit ou trousse d'antigène de VIH fourni par la société Abbott Laboratories. Pour le titrage un volume de 10 µl (provenant de chacune des alvéoles de la plaque de microtitrage) a été prédilué dans 1 ml de milieu RPMI 1640. On a fait à partir de ce stock 8 dilutions en série respectivement de 1 à 3 et de 1 à 5. Un volume de 0,1 ml de chaque dilution a été prélevé et ajouté à 0,2 ml d'une culture préincubée de cellules H-9 contenant 5 x 10⁵ cellules/ml. Après incubation à 37°C, la présence d'antigène de VIH a été testée au moyen d'un kit d'antigène de VIH fourni par la société Abbott Laboratories.

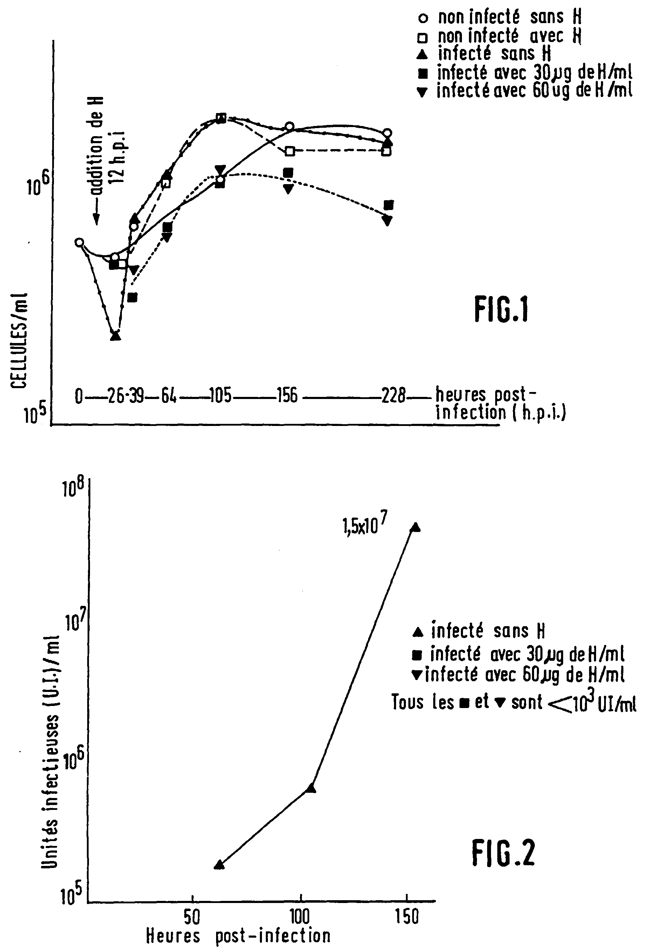

Les résultats reproduits sous forme graphique sur la figure 1 ci-annexée montrent que la flavopéreirine n'affecte pas la multiplication de cellules non infectées.

Au contraire la quantité de cellules infectées est d'environ 40% inférieure en présence de la flavopéreirine.

Ce qui est plus frappant encore, c'est que (voir la présentation des résultats sur la figure 2), alors qu'il y avait un accroissement de particules virales de cellules infectées non traitées avec le temps, on n'a pas pu, dans les limites du test appliqué, détecter la présence d'unités infectieuses au-delà de 3000 dans les séries de cellules infectées traitées avec la flavopéreirine (30 µg ou 60 µg). Cela montre que l'inhibition de l'infection dépasse au moins 99%.

Une évaluation de l'effet antiviral de la flavopéreirine a également été effectuée par étude de l'effet cytopathogène du virus de VIH sur des cellules MT4, étant donné que l'on avait observé une formation de syncytia 4 à 6 jours après l'infection par VIH 1, suivie de la mort des cellules.

La flavopéreirine a été utilisée sous la forme d'une solution alcoolique (40 mg dans 100 µl d'alcool). On a fait des dilutions dans du milieu RPMI à 10% de FCS, 1% de PSN, 1% de glutamine. Les cellules MT4 ont été mises à pré-incuber pendant deux heures à 37°C avec une dilution successive de flavopéreirine contenant 3 x 10⁵ cellules pour 10 µl de solution de flavopéreirine. On a obtenu la solution en ajoutant 100 µl d'une dilution par 10⁻⁴ du virus VIH-1, produisant une formation de syncytia en 4 à 6 jours. Après 1 heure d'incubation à 37°C, les cellules MT4 infectées ont été lavées 3 fois avec du milieu RPMI avant d'être mises en culture (3 x 10⁵ celllules/µl dans des microplaques à 24 alvéoles) en présence de différentes dilutions de flavopéreirine. Le comptage des syncytia a été effectué chaque jour en double. Les résultats sont résumés dans les tableaux I, II et III ci-dessous.

**Tableau I**

| H | d3 | | d4 | | d6 | | d7 | |
|---|---|---|---|---|---|---|---|---|
| 400 µg/ml | Tox | Tox | | | | | | |
| 100 | Tox | Tox | | | | | | |
| 50 | (+) | (+) | (+) | (+) | - | - | - | - |
| 10 | + | + | + | (+) | + | ++ | ++ | ++ |
| 1 | + | (+) | + | + | ++ | ++ | ++/T | ++/T |
| 100 ng/ml | + | + | + | + | ++ | ++ | ++/T | ++/T |
| VIH-1 seul | + | + | + | + | ++ | ++ | ++ | ++/T |
| MT4 | - | - | - | - | - | - | - | - |

**Tableau II**

| H | d3 | | d4 | | d5 | | d6 | | d7 | | d10 | |
|---|---|---|---|---|---|---|---|---|---|---|---|---|
| 100 µg/ml | Tox | Tox | | | | | | | | | | |
| 50 | - | - | - | - | - | - | - | - | - | - | - | - |
| 10 | - | - | + | (+) | + | + | + | ++ | ++ | ++ | | |
| 1 | - | - | + | + | + | ++ | ++ | ++ | ++ | ++/T | | |
| 100 ng/ml | - | (+) | + | + | + | + | ++ | ++ | ++ | ++ | | |
| VIH-1 seul | - | - | (+) | + | + | ++ | ++ | ++ | ++/T | ++/T | | |

**Tableau III**

| H | d3 | | d4 | | d5 | | d6 | | d7 | |
|---|---|---|---|---|---|---|---|---|---|---|
| 60 µg/ml | - | - | - | - | - | - | - | - | - | - |
| 30 | - | - | - | - | - | - | (+) | - | ++ | (+) |
| 10 | (+) | - | (+) | (+) | + | (+) | ++ | ++ | ++ | ++ |
| 1 | (+) | (+) | (+) | (+) | (+) | (+) | ++ | + | ++ | ++ |
| 100 ng/ml | - | (+) | (+) | (+) | + | (+) | + | ++ | ++ | ++ |
| VIH-1 seul | (+) | (+) | (+) | (+) | + | + | ++ | ++ | ++ | ++ |
| MT4 | - | - | - | - | - | - | - | - | - | - |

Le tableau I montre une toxicité cellulaire pour 100 et 400 µg/ml de flavopéreirine. A 50 µg/ml il ne se formait pas de syncytia après 7 jours de culture. De 10 µg/ml à 100 ng/ml on a observé des syncytia comme dans le VIH-1 témoin.

Le tableau II confirme la protection procurée par la flavopéreirine à 50 µg/ml et le tableau C confirme une nouvelle fois ces résultats: il ne se formait pas de syncytia à 60 µg/ml après 7 jours de culture et on en observait un peu après 2 jours avec la dose de 30 µg/ml. Essai d'infection virale (détermination de l'antigène P24)

On a inoculé 1 nanogramme d'isolats primaires de VIH-1, BRE1 (d'un patient asymptomatique) et TIG2 (d'un patient atteint du SIDA) avec 10⁶ cellules sanguines mononucléaires périphériques (PBMC) stimulées au PHA recueillies à partir de cinq donneurs négatifs pour VIH pris au hasard. Après 2 heures d'incubation, on a lavé les cellules deux fois et on les a cultivées dans 1 ml de RPMI 1640 contenant 20 UI de IL-2 par ml (Boehringer Mannheim, Allemagne), 2 µg de Polybrène (bromure d'hexadiméthrine) par ml (Sigma, St Louis, MO, USA) et 10⁻⁷ UI d'antisérum de chèvre par ml, agissant contre l'interféron humain alpha (Janssen, Beerse, Belgique). On a changé la moitié du milieu de culture après 72 heures et ensuite toutes les 48 heures jusqu'au 30^{ème} jour. On a testé les surnageants de culture par un dosage immuno-enzymatique (ELISA) quant à la production d'antigène P24 (Abbott, Chicago, IL, USA) et la densité optique (DO) de la couleur résultante a été convertie en concentration en protéine P24 par le biais d'un nomogramme standard, comme décrit par W. Lu et J.-M. Andrieu, Journal of Virology, 66(1): 334-340 (1992).

### Test d'efficacité de la flavopéreirine sur le pouvoir infectant du VIH-1

1) Première expérience: avant incubation du virus en présence de PBMC stimulées par PHA (cellules blastiques), des stocks viraux extracellulaires ont été prétraités en triple avec 30 ou 60 µg de flavopéreirine par ml pendant 2 heures.
2) Deuxième expérience: des cellules blastiques ont été prétraitées en triple avec 30 ou 60 µg de flavopéreirine par ml pendant 2 heures, et on a ensuite lavé deux fois avant d'exposer à l'inoculum viral.

### Cytotoxicité de la flavopéreirine dans les PBMC au repos et dans des cellules blastiques

Des PBMC fraîches et des PBMC blastiques prélevées sur cinq donneurs sains, séronégatifs pour VIH, pris au hasard et traités à trois reprises avec 30 ou 60 µg de flavopéreirine par ml de solution alcoolique pendant 2 heures ont été réunies. Après lavage à deux reprises, les cellules ont été mises en culture dans du milieu de culture cellulaire jusqu'au quinzième jour. La viabilité des cellules de chaque groupe a été examinée par coloration d'exclusion au bleu trypan et par analyse quantimétrique. Des cultures de PBMC séronégatives pour VIH sans flavopéreirine (composé H) ont été utilisées comme témoins.

### Inhibition de l'infection productive par VIH-1 par le composé H

1) Un prétraitement de VIH-1 par le composé H (à raison de 30 ou 60 µg/ml) a complètement inhibé l'infection des PBMC cibles par des isolats de VIH-1 primaires dérivés de patients aussi bien asymptomatiques que symptomatiques (voir tableau V plus loin).
2) Seul cependant le prétraitement des PBMC cibles avec 60 µg/ml a conduit à une inhibition complète des infections virales productives (voir tableau VI).

### Cytotoxicité du composé H dans les PBMC humaines au repos et les cellules blastiques stimulées par PHA

1) La viabilité des PBMC au repos diminuait significativement (p<0,05) dans le groupe de cellules traitées avec 60 µg/ml, mais pas dans le groupe traité avec 30 µg de composé H par ml (voir tableau VI).
2) La viabilité des cellules blastiques est apparue dépendante de l'exposition au composé H (voir tableaux IV, V et VI).

### Inhibition de l'infection productive par VIH-1 au moyen du composé H

Un prétraitement de VIH-1 avec du composé H (à raison de 10, 30, 60, 100, 200 µg/ml) inhibait l'infection des PBMC cibles par le virus d'une manière dépendante de la dose (voir tableau VII plus loin). La dose égale ou supérieure à 60 µg de composé H par ml est apparue être la concentration requise pour l'inhibition complète des infections virales productives.

Dans le système de culture de PBMC humaines primaires, l'efficacité du composé H dans l'inhibition du VIH-1 de type sauvage demeurait inchangée lorsqu'on mettait le médicament à incuber dans un milieu de culture contenant 50% de sérum humain avant l'essai d'inhibition (voir tableau VIII).

### Cytotoxicité du composé H dans les PBMC humaines stimulées par PHA

La viabilité des PBMC blastiques diminuait significativement (p<0,001) dans le groupe des cellules traitées avec 200 µg/ml, mais pas dans les groupes traités avec respectivement 100, 60, 30 et 10 µg/ml de composé H (voir tableau IX).

La réplication du VIH-1 était totalement inhibée par 60-100 µg de composé H par ml. Ces concentrations sont 2 à 4 fois plus faibles que les concentrations cytotoxiques. Un tel effet est apparu ne pas être influencé par les constituants du sérum humain.

### Effets du produit H sur la production de cytokines primaires (IL-1β et TNF-α) et secondaire (IL-6) par des monocytes normaux provenant de patients séropositifs

Les monocytes adhérents provenant du sang de deux types de donneurs ont été utilisés:
- des donneurs normaux, volontaires sains, donneurs spontanés se présentant à la banque de sang de l'hôpital de la Pitié-Salpêtrière, Paris (France);
- des donneurs séropositifs provenant de consultations médicales: ce sont des personnes en début de séroconversion et n'ayant au moment du prélèvement aucun traitement. Les facteurs de risque sont la toxicomanie par voie intraveineuse pour six d'entre eux, la transmission sexuelle pour trois patients et la transfusion sanguine (Zaire) pour un cas. Aucune corrélation entre facteurs de risques et résultats expérimentaux n'a pu être faite.

Dans le cas des donneurs séropositifs quelques anomalies ont été relevées, anomalies qualitatives touchant essentiellement les lignées polynucléaire et lymphocytaire:
- polynucléaires hypersegmentés ou hyposegmentés (de type Pegler) dans quatre cas sur 10;
- lymphocytes hyperbasophiles également dans quatre cas sur 10 (différents des précédents).

Les anomalies observées n'ont pu être corrélées avec les résultats obtenus au cours des expériences.

En analyse immunophénotypique, aucune expression des antigènes de différenciation étudiés (CD34, CD33, CD13 et CD11b) n'a été montrée déficiente.

### I. Donneurs normaux

Les dix donneurs rentrant dans ces expériences ont montré une grnde cohérence de résultats:
- aucune production spontanée ni de cytokines primaires (IL-1β et TNF-α), ni de l'interleukine-6, cytokine secondaire;
- la stimulation des monocytes pendant 48 heures avec de l'interféron-γ (1000 U/ml) n'a abouti à aucune production de cytokines primaires, sauf pour le donneur #10 en IL-β, et de cytokine secondaire, sauf pour les donneurs #1 et 5.
- les stimulations obtenues avec le LPS (lipopolysaccharide) et le mélange interféron-γ + LPS ont été comme attendues, c'est-à-dire d'amplitude différente d'un donneur à l'autre, mais toujours significatives avec un effet synergique dans le cas de la double stimulation.

### Effet du produit H

Le produit H n'a pas été utilisé dans ces expériences à plus de 20 µg/ml d'eau, valeur au-delà de laquelle une nette toxicité a pu être mise en évidence. Quatre donneurs ont été testés préalablement à 30, 50, 100 µg/ml avec une cytotoxicité pratiquement totale: plus de 40% à 30 µg/ml et 100% au-delà (les analyses des surnageants de culture n'ont pas été réalisées).

Les doses de 5, 10 et 20 µg/ml ont été choisies dès la deuxième série d'expériences, les résultats obtenus avec les deux premiers donneurs indiquant que les doses en dessous de 5 µg/ml se révélaient inactives.

### Activité directe du produit H:

- sur la production de cytokines primaires:
   . augmentation de la production de TNF-α (donneur #7) mais à une seule dose, l'effet étant également visible sur la réponse à l'IFN-γ;
   . augmentation de la production d'IL-1β (donneurs #3, 5 et 6) avec un effet-dose, mais cette réponse est restée marginale. En revanche, le donneur #10 a très bien répondu aux doses de 10 et 20 µg/ml;
- sur la production de cytokine secondaire (interleukine-6):
   . aucune modification de la réponse n'a été observée.

Activité indirecte du produit H:
- sur la production de cytokines primaires:
   . production de TNF-α: il y a eu une forte diminution de production uniquement à forte dose de produit (20 µg/ml) (p<0,05);
   . production d'IL-1β:il n'y a eu aucune modification significative des réponses au LPS ou à l'IFN-γ + LPS (p<0,05).
- sur la production de cytokine secondaire (interleukine-6):
   . il y a eu une forte diminution de la production d'IL-6; cette inhibition n'est jamais totale et, contrairement au cas du TNF-α, elle est dose-dépendante (p<0,05).

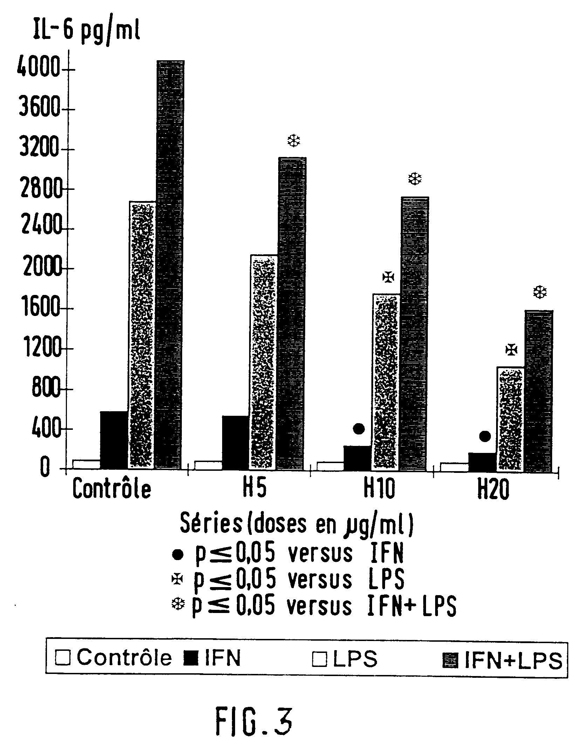

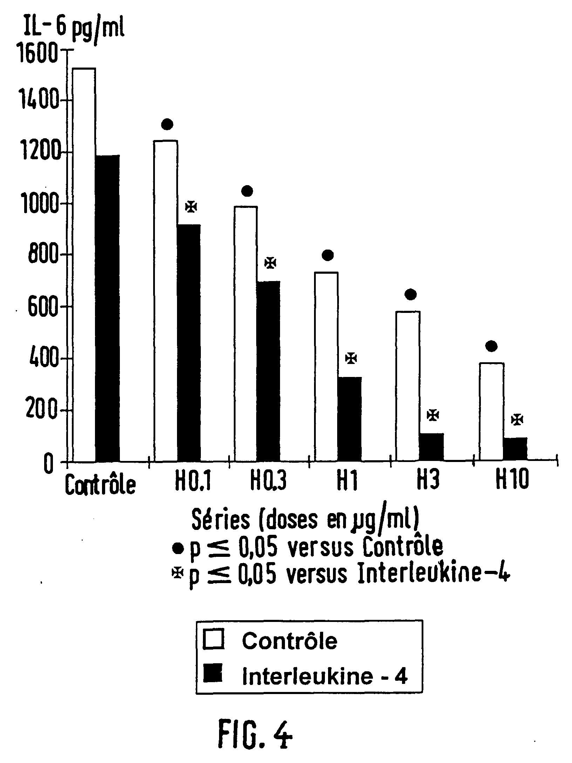

Ces différents résultats sont regroupés sur les figures 3 et 4, où les doses mesurées, en ordonnée, sont en pg/ml de cytokines, et les déviations standard ne sont pas précisées, car elles sont toujours inférieures à 10% de la moyenne.

### II. Donneurs séropositifs

La réponse des monocytes provenant des différents donneurs séropositifs a été testée en terme de réponse spontanée. Les quantités de cellules obtenues étant faibles (ce ne sont pas des donneurs prélevés par cytaphérèse), les expériences ont été limitées en fonction de ce nombre de cellules.

Activité directe du produit H:
- sur la production de cytokines primaires.

Aucune modification de la production de base n'a été observée, que ce soit pour le TNF-α ou pour l'IL-1β.
- sur la production de cytokine secondaire.

8 patients sur 10 ont présenté une réponse spontanée significative d'interleukine-6.

### Activité indirecte du produit H:

- Effet sur la production de cytokines primaires.

Les effets obtenus sont les mêmes que ceux de donneurs sains, à savoir une diminution presque totale de la production de TNF-α (uniquement à 20 µg/ml) et une absence d'effet sur la production d'interleukine-1 aux trois doses essayées (p<0,05).
- Effet sur la production d'interleukine-6.

En même temps que le produit H, l'interleukine-4 a été utilisée, car on avait montré, préalablement à ces expériences, que cette cytokine bloquait la production spontanée d'interleukine-6 chez certains patients séropositifs.

Dans cinq cas, l'interleukine-9 a été utilisée en comparaison, car elle bloque également la production d'IL-6 par des monocytes normaux stimulés par du LPS.

Les résultats obtenus sont les suivants:
. L'interleukine-4 inhibait la production d'IL-6, mais cette inhibition n'était jamais complète (p<0,05).
. L'interleukine-9 a partiellement inhibé la production spontanée (50% au maximum) et il n'y a jamais effet additif avec l'effet interleukine-4 (au contraire, dans deux cas sur cinq, il y a eu annulation des effets; ces résultats n'ont pas été reproduits sur les figures).
. Le produit H a présenté un effet inhibiteur avec un très net effet-dose (p<0,05). Il n'y a pourtant jamais eu inhibition totale de la production spontanée d'IL-6. En revanche, en présence d'interleukine-4, un effet d'amplification a pratiquement toujours été obtenu (sauf dans le cas #4), avec disparition totale de la production à 3 µg/ml de produit H (et même souvent dès 1 µg/ml) (p<0,05).

### En conclusion:

- le produit H s'est révélé toxique au-delà de la dose de 20 µg/ml in vitro sur les cellules utilisées dans le cadre des expériences du titre, à savoir des monocytes humains provenant soit de donneurs sains, soit de patients séropositifs asymptomatiques;
- le produit H s'est révélé apte à moduler la production de cytokines soit directement pour les cytokines primaires, effet qui restait faible, soit de manière très nette indirectement pour les productions de TNF-α et d'IL-6, mais non d'IL-1. De même, le produit H inhibait les productions spontanées d'IL-6 observées chez certains patients séropositifs. Cet effet inhibiteur, qui n'était jamais total dans le cadre des expériences relatées, était amplifié en présence d'IL-4;
- la normalisation des réponses IL-6 et TNF-α chez le sujet séropositif, sans pour cela inhiber les productions d'IL-1, était tout à fait importante, puisque le produit H ne modifiait pas le potentiel de relations immunes entre monocytes et lymphocytes, ainsi qu'une grande partie des réactions inflammatoires nécessaires à la survie, comme la stimulation des cellules souches de la moelle osseuse et l'établissement d'une réaction de défense au niveau général.

### Tolérance clinique et efficacité de la flavopéreirine

L'étude clinique a été effectuée sur 24 patients séropositifs présentant une numération de lymphocytes T4 comprise en valeur absolue entre 0,200 et 0,400 x 10⁹/l. Tous les patients avaient été informés sur le composé actif concerné et sur les autres médicaments antirétrovirus couramment utilisés à l'époque de l'étude. La sélection des patients a été effectuée sur la base du nombre absolu de lymphocytes T4: hommes et femmes de plus de 18 ans, ayant un indice de Karnofsky égal ou supérieur à 90%, présentant des anticorps anti-VIH-1, dans deux essais témoins successifs (méthode ELISA), des groupes CDC II, CDC III, CDC IV C2, CDC IV E de la classification CDC 87; l'hémoglobine étant supérieure à 100-120 g/l, les polynucléaires neutrophiles étant supérieurs à 1,5 x 10⁹/l, les plaquettes étant de plus de 80 x 10⁹/l, les lymphocytes T4 étant égaux ou supérieurs à 0,2 x 10⁹/l et inférieurs ou égaux à 0,4 x 10⁹/l, en l'absence de thérapie antirétrovirale, en particulier par l'AZT.

20 patients ont été admis sur la base de ces critères d'inclusion. Avant de les inclure dans l'étude, on a fait pour chacun d'eux un bilan préthérapeutique: état historique postclinique et thérapeutique, examen clinique avec détermination de la fièvre, l'anorexie et les nausées, les maux de tête, le prurit, la toux et l'expectoration, la diarrhée, les adénopathies, la mycose buccale, la dermatite séborrhéique et les lésions de Kaposi. On a également effectué une étude biologique incluant les hématies et les plaquettes, les sous-populations lymphocytaires (CD2, CD4, VD8, CD19, CD4/8), la détermination de l'antigène P24 et de la bêta-2 microglobuline, de la LDH (lactate déshydrogénase), de la ferritine plasmatique, de l'ALAT (alanine-amino-transférase), de l'ASAT (aspartate-aminotransférase) et de la créatinine plasmatique.

On a administré la flavopéreirine (composé H) sous forme de gélules de 600 mg à une dose journalière de 1-3 g, de préférence d'au moins environ 1 g de principe actif par jour. La durée moyenne du traitement était de 43 ± 11 semaines. Il y a su peu d'effets secondaires seulement pendant les trois premiers mois. On n'a observé ni toxicité sanguine ou rénale, ni modification significative dans l'ALAT ou l'ASAT. On n'a noté aucune dégradation dans la classification CDC et aucune infection; l'indice de Karnofsky se maintenait toujours autour de 100%. L'activité physique ou professionnelle des patients restait strictement normale. La réponse immunitaire au traitement était essentiellement une augmentation significative des cellules CD4+ (p<0,05), ainsi que des cellules CD19+ (p<0,05). La pente négative des CD4+ s'est inversée chez 18 patients sur 19 (p<0,05).

Tous les résultats obtenus in vitro et in vivo indiquent clairement que le composé flavopéreirine exerce un effet inhibiteur significatif sur le pouvoir infectant viral du VIH tant in vitro sur des cellules humaines que in vivo chez des patients infectés par VIH-1.

Sur 10 des malades qui ont été traités pendant 1 an, on a en outre noté les variations significatives suivantes:
- augmentation des hématies à 9 mois;
- augmentation de l'hémoglobine à 9 mois;
- augmentation de la masse totale des lymphocytes à 9 et 12 mois;
- augmentation des CD2 à 9 mois;
- augmentation des CD4 à 12 mois;
- augmentation des CD8 à 9 mois;
- augmentation des CD19 à 6-9 et 12 mois;
- augmentation des bêta-2 microglobulines à 3-6 et 12 mois.

Dans la pratique on préconise de préférence une administration par voie orale sous forme solide, telle que des comprimés ou des gélules, par exemple. Une dose unitaire d'environ 250-500 mg du principe actif est tout particulièrement appropriée.

La posologie recommandée est, compte-tenu des résultats susmentionnés et des indications de toxicité relatées, d'environ 1 à 3 g de principe actif par jour (g/d), et de préférence d'au moins environ 1 g/d, avantageusement par des prises successives réparties au cours de la journée.

Les dosages et/ou formes galéniques retenues peuvent cependant varier selon l'état du malade et le stade de l'atteinte virale à traiter. Leur adaptation au cas spécifique concerné dans chaque traitement particulier peut être réalisée aisément par l'homme du métier, sur la base de ses connaissances en la matière et si besoin est avec l'aide d'essais de routine préliminaires. A cet égard, il est tout particulièrement recommandé de prendre en compte les données fournies par une étude de pharmacocinétique faite sur l'homme pour évaluer la durée de demi-vie du principe actif administré et éventuellement d'adapter en fonction des résultats obtenus la forme de préparation pharmaceutique pour administration. Celle-ci peut ainsi comporter des formes galéniques à effet retard, par exemple.

Les doses à administrer comprennent en pratique, outre le principe actif ou un sel ou autre dérivé de celui-ci, au moins un support ou vecteur pharmaceutique et des excipients, charges, agents parfumants et/ou colorants habituels.

**Tableau IV**

| Prétraitement d'inoculum viral avec du composé H (expériences en triple) | | | | | | |
|---|---|---|---|---|---|---|
| Stock viral (1 ng/ml) | VIH prétraité avec H pendant 2 h. | Production postinfection de P24 de VIH (pg/ml) | | | | |
| | | d3 | d5 | d14 | d21 | d30 |
| VIH-1_{Asym.} (Stock_{Bret}) | Témoin | 250±25 | >1500 | | | |
| | +30 µg/ml | - | - | - | - | - |
| | +60 µg/ml | - | - | - | - | - |
| VIH-1_{SIDA} (Stock_{Tigr}) | Témoin | 575±129 | >1500 | | | |
| | +30 µg/ml | - | - | - | - | - |
| | +60 µg/ml | - | - | - | - | - |
| Cellules mononucléaires de sang périphérique (PBMC) réunies issues de cinq donneurs en bonne santé séronégatifs pour VIH choisis au hasard | | | | | | |

**Tableau V**

| Prétraitement de cellules cibles avec du composé H (expériences en triple) | | | | | | |
|---|---|---|---|---|---|---|
| Stock viral (1 ng/ml) | PBMC* prétraitées avec H pendant 2h. | Production postinfection de P24 de VIH (pg/ml) | | | | |
| | | d3 | d5 | d14 | d21 | d30 |
| VIH-1_{Asym.} (Stock_{Bret}) | Témoin | 250±25 | >1500 | | | |
| | +30 µg/ml | 113±7 | >1500 | | | |
| | +60 µg/ml | - | - | - | - | - |
| VIH-1_{SIDA} (Stock_{Tigr}) | Témoin | 575±129 | >1500 | | | |
| | +30 µg/ml | 515±103 | >1500 | | | |
| | +60 µg/ml | - | - | - | - | - |

| | | | | | | |
|---|---|---|---|---|---|---|
| *PBMC réunies à partir de cinq donneurs en bonne santé séronégatifs pour VIH choisis au hasard | | | | | | |

**Tableau VI**

| Cytotoxicité du composé H dans des PBMC humaines au repos et des PBMC stimulées par PHA (blastiques) (expériences en 5 fois) | | | | | | |
|---|---|---|---|---|---|---|
| Cellules cibles | Cellules traitées avec H (2 h.) | Viabilité (%) des PBMC après exposition au composé H | | | | |
| | | d3 | d7 | d11 | d13 | d15 |
| PBMC* | Témoin | 97±2 | 95±3 | 98±2 | 91±8 | 85±9 |
| | +30 µg/ml | 95±4 | 93±2 | 88±7 | 82±10 | 81±11 |
| | +60 µg/ml | 56±6 | 23±4 | 17±4 | 12±5 | 25±8 |
| Blastes** | Témoin | 86±5 | 84±6 | 76±4 | 75±5 | 68±7 |
| | +30 g/ml | 88±3 | 85±7 | 71±3 | 76±9 | 69±8 |
| | +60 g/ml | 79±4 | 74±5 | 70=4 | 71±8 | 63±7 |

| | | | | | | |
|---|---|---|---|---|---|---|
| * PBMC réunies provenant de cinq donneurs en bonne santé séronégatifs pour VIH choisis au hasard | | | | | | |
| ** Cellules blastiques stimulées par PHA provenant de cinq donneurs sains séronégatifs pour VIH choisis au hasard | | | | | | |

**Tableau VII**

| Prétraitement d'inoculum viral avec du composé H en l'absence de sérum humain du groupe AB (expériences en 5 fois) | | | | |
|---|---|---|---|---|
| VIH prétraité avec H pendant 2 h. | Production de P24 de VIH (pg/ml) | | | |
| | d4 | d10 | d14 | d21 |
| Témoin | 510±235 | >1500 | | |
| +200 µg/ml | - | - | - | - |
| +100 µg/ml | - | - | - | - |
| +60 µg/ml | - | - | - | - |
| +30 µg/ml | 173±102 | >1500 | | |
| +10 µg/ml | 388±124 | >1500 | | |
| Cellules mononucléaires de sang périphérique (PBMC) réunies issues de cinq donneurs en bonne santé séronégatifs pour VIH choisis au hasard | | | | |

**Tableau VIII**

| Prétraitement d'inoculum viral avec du composé H en présence de sérum humain du groupe AB (expériences on triple) | | | | | |
|---|---|---|---|---|---|
| VIH prétraité avec H pendant 2 h. | sans/avec (-/+) 50% de sérum AB | Production de P24 de VIH (pg/ml) | | | |
| | | d4 | d10 | d14 | d21 |
| Témoin | - | 510±235 | >1500 | | |
| +200 µg/ml | + | - | - | - | - |
| +100 µg/ml | + | - | - | - | - |
| +60 µg/ml | + | - | - | - | - |
| +30 µg/ml | + | 275±98 | >1500 | | |
| +10 µg/ml | + | 384±83 | >1500 | | |
| Cellules mononucléaires de sang périphérique (PBMC) réunies issues de cinq donneurs on bonne santé séronégatifs pour VIH choisis au hasard | | | | | |

**Tableau IX**

| Cytotoxicité du composé H sur des PBMC humaines stimulées par PHA (blastiques) en présence ou en l'absence de sérum humain du groupe AB (expériences on cinq fois) | | | | | |
|---|---|---|---|---|---|
| Cellules traitées avec H ( 2 h.) | sans/avec (-/+) 50% de sérum AB | Cytotoxicité du composé H après exposition à des PBMC* | | | |
| | | d4 | d10 | d14 | d21 |
| Témoin | - | 85±1,4** | 86±1,3 | 80±2,7 | 77±3,1 |
| +200 µg/ml | - | 45±6,3 | 33±7,8 | 24±7,6 | 15±5,4 |
| +100 µg/ml | - | 84±1,6 | 79±3,1 | 73±5,3 | 74±4,7 |
| +60 µg/ml | - | 86±1,5 | 82±2,4 | 82±2,6 | 80±3,3 |
| +30 µg/ml | - | 81±2,8 | 83±2,6 | 80±3,9 | 78=4,5 |
| +10 µg/ml | - | 87±1,2 | 86±1,4 | 84±1,5 | 81±2,3 |
| +200 µg/ml | + | 37±6,8 | 24±6,6 | 17±7,9 | 11±5,6 |
| +100 µg/ml | + | 83±2,3 | 80±4,5 | 77±5,6 | 73±4,4 |
| +60 µg/ml | + | 85±1,2 | 84±1,7 | 82±2,1 | 79±3,5 |
| +30 µg/ml | + | 88±1,3 | 84±2,5 | 82±3,7 | 81±4,6 |
| +10 µg/ml | + | 87±1,1 | 88±1,4 | 84±3,2 | 82±3,1 |

| | | | | | |
|---|---|---|---|---|---|
| * Cellules blastiques stimulées par PHA provenant de cinq donneurs sains séronégatifs pour VIH choisis au hasard | | | | | |
| ** Pourcentage (moyenne ± écart-type) de cellules viables | | | | | |

## Revendications

1. Utilisation de la flavopéreirine comme unique principe actif pour l'obtention d'un médicament destiné à un traitement antiviral contre le virus VIH.

2. Utilisation selon la revendication 1, caractérisée en ce que ledit principe actif est présent à raison d'environ 250-500 mg par dose unitaire.

3. Utilisation selon la revendication 2, caractérisée en ce que la flavopéreirine est présente à raison d'environ 500 mg par dose unitaire et avec indication d'une posologie de 1-3 g/d.

4. Utilisation selon la revendication 1, caractérisée en ce qu'elle comporte de la flavopéreirine comme unique principe actif, pour l'obtention d'un médicament sous forme de comprimés ou de gélules contenant par dose unitaire environ 250-500 mg de flavopéreirine ou d'un de ses sels ou autres dérivés pharmacologiquement acceptables.

5. Utilisation selon la revendication 4, caractérisée en ce que ledit médicament est sous une forme galénique à effet retard.

## Claims

1. Use of flavopereirine as the sole active ingredient for obtaining a medicine intended for an antiviral treatment against the HIV virus.

2. Use according to claim 1, characterized in that the said active ingredient is comprised in an amount of about 250-500 mg per unit dose.

3. Use according to claim 2, characterized in that the flavopereirine is comprised in an amount of about 500 mg per unit dose and with the indication of a posology of 1-3 g/d.

4. Use according to claim 1, characterized in that it comprises flavopereirine as the sole active ingredient, for obtaining a medicine having the form of tablets or capsules which comprise per unit dose about 250-500 mg of flavopereirine or of one of its salts or other pharmaceutically acceptable derivatives.

5. Use according to claim 4, characterized in that the said medicine is formulated in a time-release galenic form.

## Patentansprüche

1. Verwendung von Flavopereirin als einzigen Wirkstoff zur Herstellung eines Arzneimittels zur antiviralen Behandlung gegen HIV.

2. Verwendung nach Anspruch 1, dadurch gekennzeichnet, dass der Wirkstoff pro Einzeldosis in einer Menge von etwa 250 bis 500 mg vorliegt.

3. Verwendung nach Anspruch 2, dadurch gekennzeichnet, dass das Flavopereirin pro Einzeldosis in einer Menge von etwa 500 mg vorliegt und die Dosierung 1 bis 3 g/Tag beträgt.

4. Verwendung nach Anspruch 1, dadurch gekennzeichnet, dass sie Flavopereirin als einzigen Wirkstoff umfasst, zur Herstellung eines Arzneimittels in Form von Tabletten oder Gelatinekapseln, die pro Einzeldosis etwa 250 bis 500 mg Flavopereirin oder pharmazeutisch verträgliche Salze oder andere Derivate davon enthalten.

5. Verwendung nach Anspruch 4, dadurch gekennzeichnet, dass das Arzneimittel in einer galenischen Form mit verzögerter Wirkung vorliegt.
